# EUROPEAN PATENT SPECIFICATION

(11) **EP 1 325 755 B1**
(45) Date of publication and mention of the grant of the patent: **26.10.2005**
(21) Application number: 02017927.1
(22) Date of filing: 09.08.2002
(51) Int. Cl.: A61L 27/56, A61L 27/58

(54) **Process for preparing porous bioresorbable material having interconnected pores**
Prozess zur Herstellung poröser bioresorbierbarer Materialien mit miteinander in Verbindung stehenden Poren
Procédé de fabrication de matériaux poreux et biorésorbables avec pores interconnectés

(30) Priority: 02.01.2002 US 38419
(43) Date of publication of application: 09.07.2003
(73) Proprietor: INDUSTRIAL TECHNOLOGY RESEARCH INSTITUTE, Chutung, Hsinchu (TW)
(72) Inventor: Chen, Jui-Hsiang, Taiwan (TW); Yang, Jean-Dean, Dai-Yuan Xiang, Tao-Yuan Hsien, Taiwan (TW); Tsai, Bin-Hong, Chiautou Shiang, Kaohsiung Hsien, Taiwan (TW); Liu, Mei-Jun, Gong-Guan Xiang.Miao-Li Hsien, Taiwan (TW); Hsieh, Yu-Lin, Kaohsiung, Taiwan (TW)
(74) Representative: Merkle, Gebhard

(56) References cited:
- WO-A-00/55300
- US-A- 6 103 255

## Description

### BACKGROUND OF THE INVENTION

### 1. Field of the Invention:

The present invention relates to a process for preparing porous bioresorbable material having interconnected pores, and more particularly to a process for preparing porous bioresorbable material having interconnected pores by means of using a low molecular weight oligomer as a pore former.

### 2. Background of the Invention:

Materials that serve as analogues for a native extracellular matrix may have uses in medicine or dentistry, and may aid in the reconstruction or regeneration of bone, cartilidge, liver, skin and other tissue. The so-called bioresorbable polymers, which degrade in the body by hydrolysis into smaller molecular weight compounds that can be absorbed by biological tissues, are potential materials for fabricating such analogues. Implanting biomaterials or biodevices prepared from such bioresorbable polymers into human body decreases undesirable foreign body reaction.

Naturally occuring bioresorbable polymers include collagen, gelatin, silk, chitosan, chitin, alginate, hyaluronic acid, and chondroitin sulphate. Synthetic bioresorbable polymers include polyglycolic acid (PGA), polylactic acid (PLA), poly(glycolic-co-lactic acid (PLGA), polycaprolactone (PCL), and polydioxane. Many of the above bioresorbable polymers have been used clinically to fabricate implantable biomaterials or biodevices. For example, PGA has been used to fabricate bioresorbable suture, bioresorbable bone screw, and internal fixative devices.

In some clinical conditions, the bioresorbable polymer is fabricated into a porous matrix, also referred to as a "scaffold". Generally, cells cultured in vitro are adhered to the surface of the porous matrix and grown for a period of time. The porous matrix containing living cells is then implanted into a patient body. The implanted cells grow in the body and gradually form a tissue with specific functions, such as cartilage, bone, muscle and blood vessel.

Many processes have been proposed for fabricating a bioresorbable porous matrix, which can be classified into the following eight categories: (1) solution casting, (2) solvent-casting particulate leaching, (3) gel casting, (4) gas saturation, (5) phase separation, (6) bonded fiber, (7) particle sintering, , and (8) foaming agent.

Widmer et al. (Biomaterials, 19, p.1945-1955, 1998) and Evans et al. (Biomaterials, 20, p.1109-1115, 1999) use PLGA and PLLA polymers which were dissolved in methylene chloride. A ground salt is added to the polymer solution, stirred thoroughly, cooled, cut into small pieces, and extruded into hollow round tubes. The tubes are then cut and immersed in water for 24 hours to form porous round tubes.

Groot et al. (Biomaterials, 18, p.613-622, 1997) use 50/50 copoly(L-lactide/ε-caprolactone) to dissolve in a mixed solvent of 1,4-dioxane and c-hexane (90/10). Saccharose crystals are then added to the solution, stirred thoroughly, frozen at -15°C, evaporated under reduced pressure to remove solvent, and washed with water to remove saccharose crystals and obtain a porous material.

Ishaug-Riley et al. (Biomaterials, 19, p.1405-1412, 1998) use the solvent-casting particulate-leaching method to prepare a porous material by employing 75:25 poly(DL-lactic-co-glycolic acid) (PLGA) as the bioresorbable polymer source.

Thomson et al. (Biomaterials, 20, p.2007-2018, 1999) use the solvent-casting and salt-leaching methods to prepare a porous material by employing 85:15 poly(DL-lactic-co-glycolic acid (PLGA) as the bioresorbable polymer source.

Shalaby et al. in U.S. Patent No. 5,898,040 and No. 5,969,020 disclose a process for preparing microporous polymeric foams. A crystalline compound (m.p. is higher than 25°C) such as naphthalene, anthracene, or salicylic acid is melted. An organic crystalline polymer such as polyethylene, polypropylene, nylon 6/6, nylon 12, polyglycolic acid is contacted with the melt for a period of time, such that the crystalline compound is co-dissolved in the organic crystalline polymer. The solvent extraction or sublimation process is then performed to remove the crystalline compound from the organic crystalline polymer. A crystalline polymer material having a microporous foam structure on the surface is thus formed.

Barrows et al. in U.S. Patent No. 5,856,376 and No. 5,502,092 disclose a process for preparing a biocompatible porous matrix of bioresorbable material. A bioresorbable polymer material such as polylactic acid, polyglycolic acid, and polydioxanone and a volumetric orientation aid such as L-lactide monomer are melted. The molten mixture is then cooled to form a material having two phases. The solvent extraction process is then performed to remove the volumetric orientation aid to form a bioresorbable porous matrix.

Schindler in U.S Patent No. 4,702,917 discloses a process for preparing a porous bioresorbable polyester. Bioresorbable polymers (polycaprolactone and polyoxypropylene) are melted and then cooled to form a solidified material. The solvent extraction process is then performed to remove polyoxypropylene to form a bioresorbable porous polyester material.

Ashman in U.S. Patent No. 4,199,864 discloses a process for preparing an implantable porous film. A monomer and soluble salt (such as NaCl) crystals are mixed. Polymerization is conducted by heating. The salt crystals are then leached out with water to form a porous film.

Gogolewiski in U.S. Patent No. 4,834,747 discloses a process for preparing a multilayered material. A polymer solution near its precipitation point is first prepared. Such a polymer solution is then coated on a substrate surface. After the solvent is evaporated, a monolayered or multilayered porous material is obtained.

Bakker et al. in U.S. Patent No. 5,508,036 disclose a process for preparing a device for preventing tissue adhesion. A multilayered film having various porosity is prepared using the salt-casting method.

Mikos et al. in U.S. Patent No. 5,514,378 disclose a process for preparing a polymer membrane having a three dimensional structure. A polymer is dissolved in a solvent to form a polymer solution. Salt particles are added to the polymer solution and then poured into a mold. The polymer solution containing salt particles is heated to remove the solvent to form a polymer membrane. The polymer membrane is then placed in water or other solvent that dissolves the salt particles for a suitable time. After the salt particles are leached out, a polymer membrane having a three dimensional structure is thus prepared.

Leong in U.S. Patent No. 5,686,091 discloses a process for preparing a biodegradable foam. A biodegradable polymer is dissolved in a liquid solvent having a melting point higher than room temperature, and then cooled in a mold to form a desired shape. The solvent is sublimated under reduced pressure to obtain a biodegradable foam matrix.

Walter et al. in U.S. Patent No. 5,716,413 disclose a process for preparing a porous biodegradable implant. A gel-like biodegradable polymer is first prepared. The polymer is then kneaded to form an extensible polymeric composition, and placed into a mold to form a desired shape. After removing residual solvent, a porous biodegradable implant is thus prepared.

Healy et al. in U.S. Patent No. 5,723,508 disclose a process for preparing a porous scaffold by freeze-drying. A biodegradable polymer material such as poly(lactide/glycolide) is dissolved in a solvent to form a polymer solution. A suitable amount of water is added to the polymer solution and stirred vigorously to form an emulsion. The emulsion is quickly frozen in a mold, and then subjected to freeze-drying to remove water and the solvent to form a porous scaffold.

McGregor et al. in U.S. Patent No. 5,869,080 disclose a process for preparing a porous absorbable implant. A polymer is dispersed in a solvent or water. Frozen droplets are then added to the polymer dispersion and then frozen to form a frozen dispersion. The frozen dispersion is then subjected to freeze-drying to remove the solvent and frozen droplets to form a porous absorbable implant.

The pore morphology of a porous matrix is a key feature in its utilization. The pore of a porous matrix is preferably in an interconnected structure. With this structure, cells can be grown in the pore, and nutrients can be transferred to cells and metabolic waste can be expelled from the porous matrix both via the pore.

However, there have not been many processes proposed preparing porous bioresorbable materials having interconnected pores . There is still a need to develop a novel process for preparing a porous bioresorbable material having interconnected pores.

### SUMMARY OF THE INVENTION

The object of the present invention is to provide a process for preparing a porous bioresorbable material having interconnected pores.

To achieve the above-mentioned object, the process of the present invention includes the following steps. First, a bioresorbable polymer and a low molecular weight oligomer are dissolved in an organic solvent to form a bioresorbable polymer solution. The bioresorbable polymer has a molecular weight greater than 20,000, and the oligomer has a molecular weight of 200 to 4000. Then, the bioresorbable polymer solution is contacted with a coagulant to form a porous bioresorbable material. The low molecular weight oligomer is soluble in the coagulant, and the bioresorbable polymer is insoluble in the coagulant.

### BRIEF DESCRIPTION OF THE DRAWINGS

The present invention will become more fully understood from the detailed description given hereinbelow and the accompanying drawings, given by way of illustration only and thus not intended to be limitative of the present invention.
FIGS. 1A and 1B are schematic diagrams showing the water permeation test according to the example of the present invention, wherein the glass container is placed with the opening upward in FIG. 1A and placed upside down in FIG. 1B.

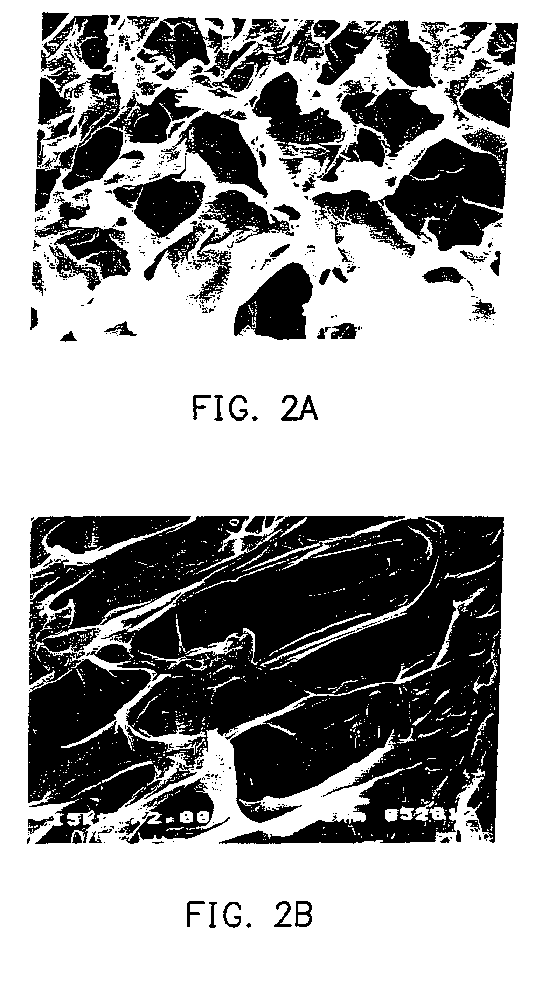

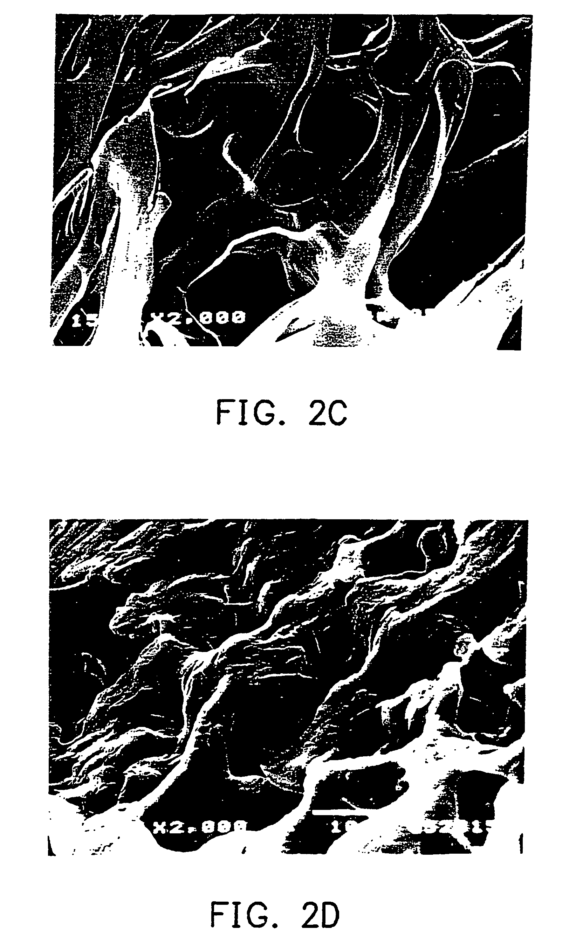

FIGS. 2A to 2D are SEM photographs of the porous PCL material obtained from Example 1 of the present invention, wherein the magnification of FIG. 2A is 750X, and the magnification of FIGS. 2B to 2D is 2000X.

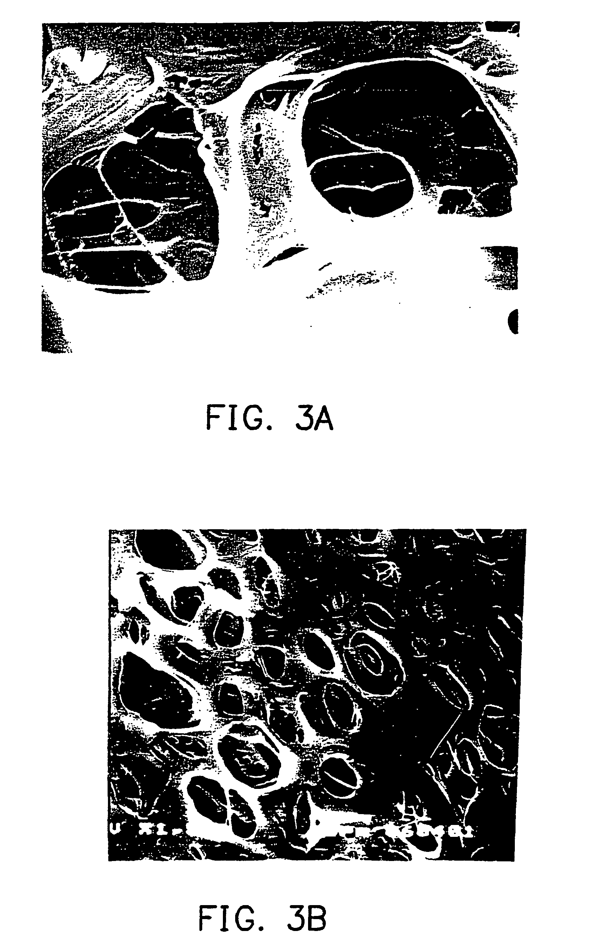

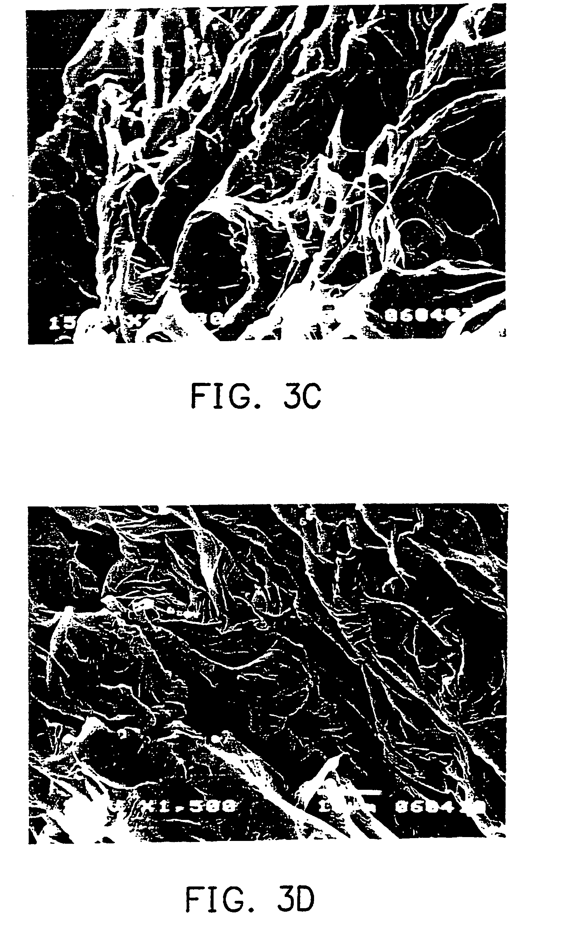

FIGS. 3A to 3D are SEM photographs of the porous PCL material obtained from Example 5 of the present invention, wherein the magnification of FIGS. 3A, 3B, 3C, and 3D is 5000X, 1500X, 2000X, and 1500X respectively.

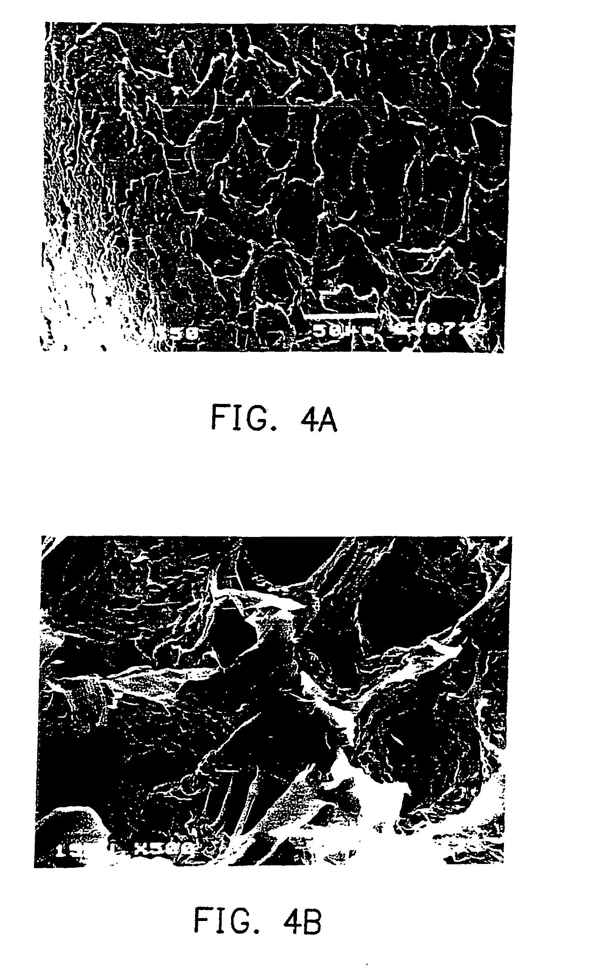

FIGS. 4A and 4B are SEM photographs of the porous PCL material obtained from Example 15 of the present invention, wherein the magnification of FIGS. 4A and 4B is 350X and 500X respectively.

### DETAILED DESCRIPTION OF THE INVENTION

The present invention provides a novel process for preparing a porous bioresorbable material having interconnected pores. First, a bioresorbable polymer and a low molecular weight oligomer are dissolved in an organic solvent to form a bioresorbable polymer solution. Then, the bioresorbable polymer solution is allowed to have a predetermined shape, for example, to form a thin film of 0.1 mm to 5 mm thick, by coating the solution on a mold surface or by pouring the solution into a container. Next, the mold coated with the bioresorbable solution or the container loaded with the bioresorbable solution is placed in a coagulant to contact with the coagulant to form a porous bioresorbable polymer material. The bioresorbable polymer solution preferably contacts the coagulant at a temperature of 5°C to 60°C, and more preferably at a temperature of 10°C to 50°C.

The mold and container can be made of any material, for example, polymer, inorganic ceramics, or metal.

The bioresorbable polymer used in the present invention can have a molecular weight higher than 20,000, and preferably ranging from 20,000 to 300,000. The low molecular weight oligomer can have a molecular weight of 200 to 4000, and preferably 300 to 3000.

According to the present invention, suitable bioresorbable polymer can be polycaprolactone (PCL), polylactic acid (PLA), polyglycolic acid (PGA), polylactic-co-glycolic acid copolymer (PLGA copolymer), polycaprolactone-polylactic acid copolymer (PCL-PLA copolymer), polycaprolactone-polyethylene glycol copolymer (PCL-PEG copolymer), or mixtures thereof.

The low molecular weight oligomer suitable for use can be bioresorbable or non-bioresorbable. Representative examples include polycaprolactone triol (PCLTL), polycaprolactone diol (PCLDL), polycaprolactone (PCL), polylactic acid (PLA), polyethylene glycol (PEG), polypropylene glycol (PPG), polytetramethylene glycol (PTMG), and mixtures thereof.

According to the present invention, the organic solvent for dissolving the bioresorbable polymer and low molecular weight oligomer can be N,N-dimethylformamide (DMF), N,N-dimethylacetamide (DMAc), THF, alcohols, chloroform, 1,4-dioxane, or mixtures thereof. The bioresorbable polymer can be present in an amount of 5-50%, more preferably 10-40%, weight fraction of the bioresorbable polymer solution. The low molecular weight oligomer can be present in an amount of 10-80% weight fraction based on the non-solvent portion of the bioresorbable polymer solution.

According to the present invention, the above coagulant preferably includes water and an organic solvent. The organic solvent in the coagulant can be present in an amount of 5-60%, and preferably 10-50% weight fraction. The organic solvent in the coagulant can be amides, ketones, alcohols, or mixtures thereof. Preferably, the organic solvent in the coagulant includes a ketone and an alcohol.

Representative examples of the organic solvent in the coagulant include N,N-dimethylformamide (DMF), N,N-dimethylacetamide (DMAc), ketones such as acetone and methyl ethyl ketone (MEK), and alcohols such as methanol, ethanol, propanol, isopropanol, and butanol.

In the present invention, the organic solvent used for preparing the bioresorbable polymer solution is a good solvent to the bioresorbable polymer. The organic solvent in the bioresorbable polymer solution exchanges with the bad solvent in the coagulant through diffusion. Thus, the polymer material gradually precipitates to form a matrix with certain foaming extent. This is the so-called phase separation method. Conventionally, the material formed only by exchange between good solvent and bad solvent has low porosity and is non-uniform. Also, the pores are in a form of non-interconnected closed cell.

However, the present invention not only uses the phase separation method, but also uses a low molecular weight oligomer. The main feature of the present invention is that a low molecular weight oligomer is added to a bioresorbable polymer solution. Since oligomer has a considerable molecular weight, it diffuses into the coagulant at a slower rate in the precipiation process of the bioresorbable polymer solution. In this manner, a porous bioresorbable material having uniform interconnected pores is formed. Therefore, the low molecular weight oligomer acts as a pore former in the present invention. The porosity and pore size of the finally-formed porous material can be adjusted by means of choosing the species and molecular weight of the low molecular weight oligomer and the content in the bioresorbable polymer solution.

After the bioresorbable polymer solution contacts the coagulant, the obtained porous bioresorbable material is preferably placed in a washing liquid in order to remove the oligomer. The washing liquid can include water and an organic solvent such as ketones, alcohols, or mixtures thereof. Representative examples of the ketone include acetone and methyl ethyl ketone (MEK). Representative examples of the alcohol include methanol, ethanol, propanol, isopropanol and butanol.

The following examples are intended to illustrate the process and the advantages of the present invention more fully without limiting its scope, since numerous modifications and variations will be apparent to those skilled in the art.

### Example 1

15 g of polycaprolactone (PCL) having a molecular weight about 80,000 and 15 g of polyethylene glycol (PEG) having a molecular weight of 1000 (an oligomer) were added to 70 g of THF, which was stirred thoroughly at room temperature to form a PCL solution containing PEG oligomer. The solution was then coated onto the surface of a plate-shaped mold to a thickness of about 0.5 mm. The plate-shaped mold coated with PCL solution was then placed in a coagulant at 25°C (the composition of the coagulant and coagulating time are shown in Table 1). Thus, the PCL solution was coagulated to form a porous PCL material. The porous PCL material was then immersed in a 50% acetone solution (washing liquid) for 2 hours, and then washed with clean water and dried to obtain the final flat film-shaped porous PCL material.

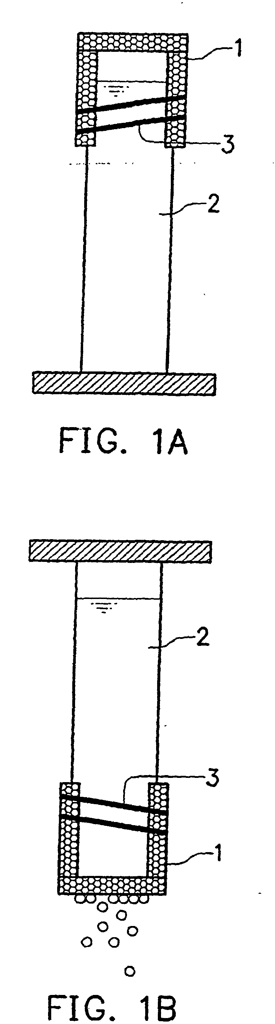

The following procedures were conducted in order to determine whether the flat film-shaped porous PCL material has an interconnected pore structure. Referring to FIG. 1A, the flat film-shaped porous PCL material 1 was covered over a glass container 2 loaded with water to seal the container 2. The PCL material 1 was fixed to the container 2 with, for example, a rubber band 3. Then, the container 2 was turned upside down as shown in FIG. 1B. After a few seconds, water in the container 2 gradually penetrated through the porous PCL material 1. Such a water penetration test proved that the obtained PCL flat film had interconnected pores.

Specimens 1A, 1B, 1C, and 1D were observed by SEM (scanning electron microscope) to doubly assure that the PCL flat film obtained was a material having an interconnected pore structure.

**Table 1**

| Specimen | Coagulant | Coagulating time (hr) | Porous structure of porous matrix | SEM photo |
|---|---|---|---|---|
| 1A | 40 wt% acetone | 4 | interconnected | FIG. 2A |
| 1B | 40 wt% ethanol | 4 | interconnected | FIG. 2B |
| 1C | 60 wt% ethanol | 4 | interconnected | FIG. 2C |
| 1D | 20 wt% DMF | 4 | interconnected | FIG. 2D |

### Example 2

15 g of polycaprolactone (PCL) having a molecular weight about 80,000 and 15 g of polypropylene glycol (PEG) having a molecular weight of 1000 (an oligomer) were added to 70 g of THF, which was stirred thoroughly at room temperature to form a PCL solution. The solution was then coated onto the surface of a plate-shaped mold to a thickness of about 0.5 mm. The plate-shaped mold coated with PCL solution was then placed in a coagulant at 25°C (the composition of the coagulant and coagulating time are shown in Table 2). Thus, the PCL solution was coagulated to form a porous PCL material. The porous PCL material was then immersed in a 50% acetone solution (washing liquid) for 2 hours, and then washed with clean water and dried to obtain the final flat film-shaped porous PCL material. The flat film-shaped porous PCL material obtained was tested by the water permeation test to confirm that the PCL flat film was a material having an interconnected pore structure.

**Table 2**

| Specimen | Coagulant | Coagulating time (hr) | Pore structure of porous matrix |
|---|---|---|---|
| 2A | 40 wt% acetone | 3 | interconnected |
| 2B | 40 wt% ethanol | 3 | interconnected |
| 2C | 60 wt% ethanol | 3 | interconnected |
| 2D | 20 wt% DMF | 3 | interconnected |

### Example 3

15 g of polycaprolactone (PCL) having a molecular weight about 80,000 and 15 g of polytetramethylene glycol (PTMG) having a molecular weight of 1000 (an oligomer) were added to 70 g of THF, which was stirred thoroughly at room temperature to form a PCL solution. The solution was then coated onto the surface of a plate-shaped mold to a thickness of about 0.5 mm. The plate-shaped mold coated with PCL solution was then placed in a coagulant at 25°C (the composition of the coagulant and coagulating time are shown in Table 3). Thus, the PCL solution was coagulated to form a porous PCL material. The porous PCL material was then immersed in a 50% acetone solution (washing liquid) for 2 hours, and then washed with clean water and dried to obtain the final flat film-shaped porous PCL material. The flat film-shaped porous PCL material obtained was tested by the water permeation test to confirm that the PCL flat film was a material having an interconnected pore structure.

**Table 3**

| Specimen | Coagulant | Coagulating time (hr) | Pore structure of porous matrix |
|---|---|---|---|
| 3A | 40 wt% acetone | 2 | interconnected |
| 3B | 40 wt% ethanol | 2 | interconnected |
| 3C | 60 wt% ethanol | 2 | interconnected |
| 3D | 20 wt% DMF | 2 | interconnected |

### Example 4

15 g of polycaprolactone (PCL) having a molecular weight about 80,000 and 15 g of polycaprolactone triol (PCLTL) having a molecular weight of 300 (an oligomer) were added to 70 g of THF, which was stirred thoroughly at room temperature to form a PCL solution. The solution was then coated onto the surface of a plate-shaped mold to a thickness of about 0.5 mm. The plate-shaped mold coated with PCL solution was then placed in a coagulant at 25°C (the composition of the coagulant and coagulating time are shown in Table 4). Thus, the PCL solution was coagulated to form a porous PCL material. The porous PCL material was then immersed in a 50% acetone solution (washing liquid) for 2 hours, and then washed with clean water and dried to obtain the final flat film-shaped porous PCL material. The flat film-shaped porous PCL material obtained was tested by the water permeation test to confirm that the PCL flat film was a material having an interconnected pore structure.

**Table 4**

| Specimen | Coagulant | Coagulating time (hr) | Pore structure of porous matrix |
|---|---|---|---|
| 4A | 40 wt% acetone | 4 | interconnected |
| 4B | 40 wt% ethanol | 4 | interconnected |
| 4C | 60 wt% ethanol | 4 | interconnected |
| 4D | 20 wt% DMF | 4 | interconnected |

### Example 5

15 g of polycaprolactone (PCL) having a molecular weight about 80,000 and 15 g of polyethylene glycol (PEG) having a molecular weight of 1000 (an oligomer) were added to 70 g of DMF, which was stirred thoroughly at room temperature to form a PCL solution. The solution was then coated onto the surface of a plate-shaped mold to a thickness of about 0.4 mm. The plate-shaped mold coated with PCL solution was then placed in a coagulant at 20°C (the composition of the coagulant and coagulating time are shown in Table 5). Thus, the PCL solution was coagulated to form a porous PCL material. The porous PCL material was then immersed in a 50% acetone solution (washing liquid) for 2 hours, and then washed with clean water and dried to obtain the final flat film-shaped porous PCL material. The flat film-shaped porous PCL material obtained was tested by the water permeation test to confirm that the PCL flat film was a material having an interconnected pore structure.

Specimens 5A, 5B, 5C, and 5D were observed by SEM (scanning electron microscope) to doubly assure that the PCL flat film obtained was a material having an interconnected pore structure.

**Table 5**

| Specimen | Coagulant | Coagulating time (hr) | Porous structure of porous matrix | SEM photo |
|---|---|---|---|---|
| 5A | 40 wt% acetone | 3 | interconnected | FIG. 3A |
| 5B | 40 wt% ethanol | 3 | interconnected | FIG. 3B |
| 5C | 60 wt% ethanol | 3 | interconnected | FIG. 3C |
| 5D | 20 wt% DMF | 3 | interconnected | FIG. 3D |

### Example 6

15 g of polycaprolactone (PCL) having a molecular weight about 80,000 and 15 g of polypropylene glycol (PPG) having a molecular weight of 1000 (an oligomer) were added to 70 g of DMF, which was stirred thoroughly at room temperature to form a PCL solution. The solution was then coated onto the surface of a plate-shaped mold to a thickness of about 0.4 mm. The plate-shaped mold coated with PCL solution was then placed in a coagulant at 20°C (the composition of the coagulant and coagulating time are shown in Table 6). Thus, the PCL solution was coagulated to form a porous PCL material. The porous PCL material was then immersed in a 50% acetone solution (washing liquid) for 2 hours, and then washed with clean water and dried to obtain the final flat film-shaped porous PCL material. The flat film-shaped porous PCL material obtained was tested by the water permeation test to confirm that the PCL flat film was a material having an interconnected pore structure.

**Table 6**

| Specimen | Coagulant | Coagulating time (hr) | Pore structure of porous matrix |
|---|---|---|---|
| 6A | 40 wt% acetone | 2 | interconnected |
| 6B | 40 wt% ethanol | 2 | interconnected |
| 6C | 60 wt% ethanol | 2 | interconnected |
| 6D | 20 wt% DMF | 2 | interconnected |

### Example 7

15 g of polycaprolactone (PCL) having a molecular weight about 80,000 and 15 g of polytetramethylene glycol (PTMG) having a molecular weight of 1000 (an oligomer) were added to 70 g of DMF, which was stirred thoroughly at room temperature to form a PCL solution. The solution was then coated onto the surface of a plate-shaped mold to a thickness of about 0.4 mm. The plate-shaped mold coated with PCL solution was then placed in a coagulant at 20°C (the composition of the coagulant and coagulating time are shown in Table 7). Thus, the PCL solution was coagulated to form a porous PCL material. The porous PCL material was then immersed in a 50% acetone solution (washing liquid) for 2 hours, and then washed with clean water and dried to obtain the final flat film-shaped porous PCL material. The flat film-shaped porous PCL material obtained was tested by the water permeation test to confirm that the PCL flat film was a material having an interconnected pore structure.

**Table 7**

| Specimen | Coagulant | Coagulating time (hr) | Pore structure of porous matrix |
|---|---|---|---|
| 7A | 40 wt% acetone | 4 | interconnected |
| 7B | 40 wt% ethanol | 4 | interconnected |
| 7C | 60 wt% ethanol | 4 | interconnected |
| 7D | 20 wt% DMF | 4 | interconnected |

### Example 8

15 g of polycaprolactone (PCL) having a molecular weight about 80,000 and 15 g of polycaprolactone triol (PCLTL) having a molecular weight of 300 (an oligomer) were added to 70 g of DMF, which was stirred thoroughly at room temperature to form a PCL solution. The solution was then coated onto the surface of a plate-shaped mold to a thickness of about 0.2 mm. The plate-shaped mold coated with PCL solution was then placed in a coagulant at 20°C (the composition of the coagulant and coagulating time are shown in Table 8). Thus, the PCL solution was coagulated to form a porous PCL material. The porous PCL material was then immersed in a 50% acetone solution (washing liquid) for 2 hours, and then washed with clean water and dried to obtain the final flat film-shaped porous PCL material. The flat film-shaped porous PCL material obtained was tested by the water permeation test to confirm that the PCL flat film was a material having an interconnected pore structure.

**Table 8**

| Specimen | Coagulant | Coagulating time (hr) | Pore structure of porous matrix |
|---|---|---|---|
| 8A | 40 wt% acetone | 1 | interconnected |
| 8B | 40 wt% ethanol | 1 | interconnected |
| 8C | 60 wt% ethanol | 1 | interconnected |
| 8D | 20 wt% DMF | 1 | interconnected |

### Example 9

15 g of polycaprolactone (PCL) having a molecular weight about 80,000 and 15 g of polycaprolactone diol (PCLDL) having a molecular weight of 1250 (an oligomer) were added to 70 g of DMF, which was stirred thoroughly at room temperature to form a PCL solution. The solution was then coated onto the surface of a plate-shaped mold to a thickness of about 0.4 mm. The plate-shaped mold coated with PCL solution was then placed in a coagulant at 20°C (the composition of the coagulant and coagulating time are shown in Table 9). Thus, the PCL solution was coagulated to form a porous PCL material. The porous PCL material was then immersed in a 50% acetone solution (washing liquid) for 2 hours, and then washed with clean water and dried to obtain the final flat film-shaped porous PCL material. The flat film-shaped porous PCL material obtained was tested by the water permeation test to confirm that the PCL flat film was a material having an interconnected pore structure.

**Table 9**

| Specimen | Coagulant | Coagulating time (hr) | Pore structure of porous matrix |
|---|---|---|---|
| 9A | 40 wt% acetone | 4 | interconnected |
| 9B | 40 wt% ethanol | 4 | interconnected |
| 9C | 60 wt% ethanol | 4 | interconnected |
| 9D | 20 wt% DMF | 4 | interconnected |

### Example 10

15 g of polycaprolactone (PCL) having a molecular weight about 80,000 and 15 g of polycaprolactone diol (PCLDL) having a molecular weight of 1250 (an oligomer) were added to 70 g of THF, which was stirred thoroughly at room temperature to form a PCL solution. The solution was then coated onto the surface of a plate-shaped mold to a thickness of about 4 mm. The plate-shaped mold coated with PCL solution was then placed in a coagulant at 20°C (the composition of the coagulant and coagulating time are shown in Table 10). Thus, the PCL solution was coagulated to form a porous PCL material. The porous PCL material was then immersed in a 50% acetone solution (washing liquid) for 24 hours, and then washed with clean water and dried to obtain the final flat film-shaped porous PCL material. The flat film-shaped porous PCL material obtained was tested by the water permeation test to confirm that the PCL flat film was a material having an interconnected pore structure.

**Table 10**

| Specimen | Coagulant | Coagulating time (hr) | Pore structure of porous matrix |
|---|---|---|---|
| 10A | 40 wt% acetone | 24 | interconnected |
| 10B | 40 wt% ethanol | 24 | interconnected |
| 10C | 20 wt% DMF | 24 | interconnected |

### Example 11

15 g of polycaprolactone (PCL) having a molecular weight about 80,000 and 15 g of polyethylene glycol (PEG) having a molecular weight of 1250 (an oligomer) were added to 70 g of THF, which was stirred thoroughly at room temperature to form a PCL solution. The solution was then coated onto the surface of a plate-shaped mold to a thickness of about 4 mm. The plate-shaped mold coated with PCL solution was then placed in a coagulant at 20°C (the composition of the coagulant and coagulating time are shown in Table 11). Thus, the PCL solution was coagulated to form a porous PCL material. The porous PCL material was then immersed in a 40% acetone solution (washing liquid) for 24 hours, and then washed with clean water and dried to obtain the final flat film-shaped porous PCL material. The flat film-shaped porous PCL material obtained was tested by the water permeation test to confirm that the PCL flat film was a material having an interconnected pore structure.

**Table 11**

| Specimen | Coagulant | Coagulating time (hr) | Pore structure of porous matrix |
|---|---|---|---|
| 11A | 40 wt% ethanol | 24 | interconnected |

### Example 12

15 g of polycaprolactone (PCL) having a molecular weight about 80,000, 7 g of polycaprolactone triol (PCLTL) having a molecular weight of 300 (an oligomer), and 8 g of polyethylene glycol (PEG) having a molecular weight of 300 were added to 55 g of DMF, which was stirred thoroughly at room temperature to form a PCL solution. The solution was then coated onto the surface of a plate-shaped mold to a thickness of about 0.4 mm. The plate-shaped mold coated with PCL solution was then placed in a coagulant at 25°C (the composition of the coagulant and coagulating time are shown in Table 12). Thus, the PCL solution was coagulated to form a porous PCL material. The porous PCL material was then immersed in a 40% acetone solution (washing liquid) for 8 hours, and then washed with clean water and dried to obtain the final flat film-shaped porous PCL material. The flat film-shaped porous PCL material obtained was tested by the water permeation test to confirm that the PCL flat film was a material having an interconnected pore structure.

**Table 12**

| Specimen | Coagulant | Coagulating time (hr) | Pore structure of porous matrix |
|---|---|---|---|
| 12A | 40 wt% acetone | 3 | interconnected |
| 12B | 40 wt% ethanol | 3 | interconnected |
| 12C | 20 wt% DMF | 3 | interconnected |

### Example 13

15 g of polycaprolactone (PCL) having a molecular weight about 80,000 and 15 g of polycaprolactone triol (PCLTL) having a molecular weight of 300 (an oligomer) were added to a mixed organic solvent containing 35 g of DMF and 35 g of THF, which was stirred thoroughly at room temperature to form a PCL solution. The solution was then coated onto the surface of a plate-shaped mold to a thickness of about 0.4 mm. The plate-shaped mold coated with PCL solution was then placed in a coagulant at 25°C (the composition of the coagulant and coagulating time are shown in Table 13). Thus, the PCL solution was coagulated to form a porous PCL material. The porous PCL material was then immersed in a 40% acetone solution (washing liquid) for 8 hours, and then washed with clean water and dried to obtain the final flat film-shaped porous PCL material. The flat film-shaped porous PCL material obtained was tested by the water permeation test to confirm that the PCL flat film was a material having an interconnected pore structure.

**Table 13**

| Specimen | Coagulant | Coagulating time (hr) | Pore structure of porous matrix |
|---|---|---|---|
| 13A | 40 wt% acetone | 4 | interconnected |
| 13B | 40 wt% ethanol | 4 | interconnected |
| 13C | 20 wt% DMF | 4 | interconnected |

### Example 14

15 g of polycaprolactone (PCL) having a molecular weight about 80,000 and 15 g of polycaprolactone triol (PCLTL) having a molecular weight of 300 (an oligomer) were added to a mixed organic solvent containing 55 g of DMF and 15 g of ethanol, which was stirred thoroughly at room temperature to form a PCL solution. The solution was then coated onto the surface of a plate-shaped mold to a thickness of about 0.4 mm. The plate-shaped mold coated with PCL solution was then placed in a coagulant at 25°C (the composition of the coagulant and coagulating time are shown in Table 14). Thus, the PCL solution was coagulated to form a porous PCL material. The porous PCL material was then immersed in a 40% acetone solution (washing liquid) for 8 hours, and then washed with clean water and dried to obtain the final flat film-shaped porous PCL material. The flat film-shaped porous PCL material obtained was tested by the water permeation test to confirm that the PCL flat film was a material having an interconnected pore structure.

**Table 14**

| Specimen | Coagulant | Coagulating time (hr) | Pore structure of porous matrix |
|---|---|---|---|
| 14A | 40 wt% acetone | 4 | interconnected |
| 14B | 40 wt% ethanol | 4 | interconnected |
| 14C | 20 wt% DMF | 4 | interconnected |

### Example 15

15 g of polycaprolactone (PCL) having a molecular weight about 80, 000 and 10 g of polycaprolactone triol (PCLTL) having a molecular weight of 300 (an oligomer) were added to 75 g of THF, which was stirred thoroughly to form a PCL solution labeled to 15A. 15 g of PCL having a molecular weight about 80,000 and 20 g of PCLTL having a molecular weight of 300 (an oligomer) were added to 65 g of THF, which was stirred thoroughly to form a PCL solution labeled to 15B. 15 g of PCL having a molecular weight about 80, 000 and 30 g of PCLTL having a molecular weight of 300 (an oligomer) were added to 45 g of THF, which was stirred thoroughly to form a PCL solution labeled to 15C. Each solution was then coated onto the surface of a plate-shaped mold to a thickness of about 0.4 mm. The plate-shaped mold coated with PCL solution was then placed in a coagulant at 25°C (the composition of the coagulant and coagulating time are shown in Table 15). Thus, the PCL solution was coagulated to form a porous PCL material. The porous PCL material was then immersed in a 40% acetone solution (washing liquid) for 12 hours, and then washed with clean water and dried to obtain the final flat film-shaped porous PCL material. The flat film-shaped porous PCL materials (15A, 15B, and 15C) obtained were tested by the water permeation test to confirm that the PCL flat films were materials having an interconnected pore structure.

**Table 15**

| Specimen | Coagulant | Coagulating time (hr) | Porous structure of porous matrix | SEM photo |
|---|---|---|---|---|
| 15A | 40 wt% acetone | 12 | interconnected | -- |
| 15B | 40 wt% acetone | 12 | interconnected | FIG. 4A |
| 15C | 40 wt% acetone | 12 | interconnected | FIG. 4B |

### Example 16

15 g of polycaprolactone (PCL) having a molecular weight about 80,000 and 30 g of polycaprolactone triol (PCLTL) having a molecular weight of 300 (an oligomer) were added to 45 g of DMF, which was stirred thoroughly at room temperature to form a PCL solution. The solution was then coated onto the surface of a plate-shaped mold to a thickness of about 0.4 mm. The plate-shaped mold coated with PCL solution was then placed in a coagulant at 25°C (the composition of the coagulant and coagulating time are shown in Table 16). Thus, the PCL solution was coagulated to form a porous PCL material. The porous PCL material was then immersed in a 40% acetone solution (washing liquid) for 12 hours, and then washed with clean water and dried to obtain the final flat film-shaped porous PCL material. The flat film-shaped porous PCL material obtained was tested by the water permeation test to confirm that the PCL flat film was a material having an interconnected pore structure.

**Table 16**

| Specimen | Coagulant | Coagulating time (hr) | Pore structure of porous matrix |
|---|---|---|---|
| 16A | 40 wt% acetone | 6 | interconnected |
| 16B | 40 wt% ethanol | 6 | interconnected |
| 16C | 20 wt% DMF | 6 | interconnected |

### Example 17

15 g of polycaprolactone (PCL) having a molecular weight about 80, 000 and 30 g of polycaprolactone triol (PCLTL) having a molecular weight of 300 (an oligomer) were added to 45 g of THF, which was stirred thoroughly at room temperature to form a PCL solution. The solution was then coated onto the surface of a plate-shaped mold to a thickness of about 0.4 mm. The plate-shaped mold coated with PCL solution was then placed in a coagulant at 25°C (the composition of the coagulant and coagulating time are shown in Table 17). Thus, the PCL solution was coagulated to form a porous PCL material. The porous PCL material was then immersed in a 40% acetone solution (washing liquid) for 12 hours, and then washed with clean water and dried to obtain the final flat film-shaped porous PCL material. The flat film-shaped porous PCL material obtained was tested by the water permeation test to confirm that the PCL flat film was a material having an interconnected pore structure.

**Table 17**

| Specimen | Coagulant | Coagulating time (hr) | Pore structure of porous matrix |
|---|---|---|---|
| 17A | 40 wt% acetone | 6 | interconnected |
| 17B | 40 wt% ethanol | 6 | interconnected |
| 17C | 20 wt% DMF | 6 | interconnected |

### Example 18

30 g of polycaprolactone (PCL) having a molecular weight about 30,000 and 15 g of polycaprolactone triol (PCLTL) having a molecular weight of 300 (an oligomer) were added to 55 g of DMF, which was stirred thoroughly at room temperature to form a PCL solution. The solution was then coated onto the surface of a plate-shaped mold to a thickness of about 0.4 mm. The plate-shaped mold coated with PCL solution was then placed in a coagulant at 25°C (the composition of the coagulant and coagulating time are shown in Table 18). Thus, the PCL solution was coagulated to form a porous PCL material. The porous PCL material was then immersed in a 50% acetone solution (washing liquid) for 6 hours, and then washed with clean water and dried to obtain the final flat film-shaped porous PCL material. The flat film-shaped porous PCL material obtained was tested by the water permeation test to confirm that the PCL flat film was a material having an interconnected pore structure.

**Table 18**

| Specimen | Coagulant | Coagulating time (hr) | Pore structure of porous matrix |
|---|---|---|---|
| 18A | 40 wt% acetone | 8 | interconnected |
| 18B | 40 wt% ethanol | 8 | interconnected |
| 18C | 20 wt% DMF | 8 | interconnected |

### Example 19

30 g of 75/25 PCL-PLA copolymer (polycaprolactone-polylactic acid copolymer) (a bioresorbable polymer) and 15 g of polycaprolactone triol (PCLTL) having a molecular weight of 300 (an oligomer) were added to 55 g of THF, which was stirred thoroughly at room temperature to form a PCL-PLA solution. The solution was then coated onto the surface of a plate-shaped mold to a thickness of about 0.4 mm. The plate-shaped mold coated with PCL-PLA solution was then placed in a coagulant at 25°C (the composition of the coagulant and coagulating time are shown in Table 19). Thus, the PCL-PLA solution was coagulated to form a porous PCL-PLA material. The porous PCL-PLA material was then immersed in a 40% acetone solution (washing liquid) for 12 hours, and then washed with clean water and dried to obtain the final flat film-shaped porous PCL-PLA material. The flat film-shaped porous PCL-PLA material obtained was tested by the water permeation test to confirm that the PCL-PLA flat film was a material having an interconnected pore structure.

**Table 19**

| Specimen | Coagulant | Coagulating time (hr) | Pore structure of porous matrix |
|---|---|---|---|
| 19A | 40 wt% acetone | 12 | interconnected |
| 19B | 40 wt% ethanol | 12 | interconnected |
| 19C | 20 wt% DMF | 12 | interconnected |

### Example 20

30 g of polylactic acid (PLA) and 15 g of polycaprolactone triol (PCLTL) having a molecular weight of 300 (an oligomer) were added to 55 g of THF, which was stirred thoroughly at room temperature to form a PLA solution. The solution was then coated onto the surface of a plate-shaped mold to a thickness of about 0.4 mm. The plate-shaped mold coated with PLA solution was then placed in a coagulant at 25°C (the composition of the coagulant and coagulating time are shown in Table 20). Thus, the PLA solution was coagulated to form a porous PLA material. The porous PLA material was then immersed in a 40% acetone solution (washing liquid) for 12 hours, and then washed with clean water and dried to obtain the final flat film-shaped porous PLA material. The flat film-shaped porous PLA material obtained was tested by the water permeation test to confirm that the PLA flat film was a material having an interconnected pore structure.

**Table 20**

| Specimen | Coagulant | Coagulating time (hr) | Pore structure of porous matrix |
|---|---|---|---|
| 20A | 40 wt% acetone | 12 | interconnected |
| 20B | 40 wt% ethanol | 12 | interconnected |
| 20C | 20 wt% DMF | 12 | interconnected |

### Example 21

30 g of poly-lactic-co-glycolic acid copolymer (PLGA) and 15 g of polycaprolactone triol (PCLTL) having a molecular weight of 300 (an oligomer) were added to 55 g of THF, which was stirred thoroughly at room temperature to form a PLGA solution. The solution was then coated onto the surface of a plate-shaped mold to a thickness of about 0.4 mm. The plate-shaped mold coated with PLGA solution was then placed in a coagulant at 25°C (the composition of the coagulant and coagulating time are shown in Table 21). Thus, the PLGA solution was coagulated to form a porous PLGA material. The porous PLGA material was then immersed in a 40% acetone solution (washing liquid) for 12 hours, and then washed with clean water and dried to obtain the final flat film-shaped porous PLGA material. The flat film-shaped porous PLGA material obtained was tested by the water permeation test to confirm that the PLGA flat film was a material having an interconnected pore structure.

**Table 21**

| Specimen | Coagulant | Coagulating time (hr) | Pore structure of porous matrix |
|---|---|---|---|
| 21A | 40 wt% acetone | 12 | interconnected |
| 21B | 40 wt% ethanol | 12 | interconnected |
| 21C | 20 wt% DMF | 12 | interconnected |

The foregoing description of the preferred embodiments of this invention has been presented for purposes of illustration and description. Obvious modifications or variations are possible in light of the above teaching. The embodiments chosen and described provide an excellent illustration of the principles of this invention and its practical application to thereby enable those skilled in the art to utilize the invention in various embodiments and with various modifications as are suited to the particular use contemplated. All such modifications and variations are within the scope of the present invention as determined by the appended claims when interpreted in accordance with the breadth to which they are fairly, legally, and equitably entitled.

## Claims

1. A process for preparing a porous bioresorbable material having interconnected pores, comprising the following steps:
dissolving a bioresorbable polymer and a low molecular weight oligomer in an organic solvent to form a bioresorbable polymer solution, wherein the bioresorbable polymer has a molecular weight greater than 20,000, and the oligomer has a molecular weight of 200 to 4000; and
contacting the bioresorbable polymer solution with a coagulant to form the porous bioresorbable material, wherein the low molecular weight oligomer is soluble in the coagulant, and the bioresorbable polymer is insoluble in the coagulant.

2. The process as claimed in claim 1, wherein, before the bioresorbable polymer solution is contacted with the coagulant, the solution is caused to have a predetermined shape.

3. The process as claimed in claim 2, wherein the step of causing the solution to have a predetermined shape comprises coating the solution onto a mold surface.

4. The process as claimed in claim 2, wherein the step of causing the solution to have a predetermined shape comprises pouring the solution into a container.

5. The process as claimed in claim 1, wherein the bioresorbable polymer has a molecular weight of 20,000 to 300,000.

6. The process as claimed in claim 1, wherein the bioresorbable polymer is selected from the group consisting of polycaprolactone (PCL), polylactic acid (PLA), polyglycolic acid (PGA), poly-lactic-co-glycolic acid copolymer (PLGA copolymer), polycaprolactone-polylactic acid copolymer (PCL-PLA copolymer), polycaprolactone-polyethylene glycol copolymer (PCL-PEG copolymer), and mixtures thereof.

7. The process as claimed in claim 1, wherein the low molecular weight oligomer has a molecular weight of 300 to 3000.

8. The process as claimed in claim 1, wherein the low molecular weight oligomer is selected from the group consisting of polycaprolactone triol (PCLTL), polycaprolactone diol (PCLDL), polycaprolactone (PCL), polylactic acid (PLA), polyethylene glycol (PEG), polypropylene glycol (PPG), polytetramethylene glycol (PTMG), and mixtures thereof.

9. The process as claimed in claim 1, wherein the organic solvent for dissolving the bioresorbable polymer and low molecular weight oligomer is selected from the group consisting of N,N-dimethylformamide (DMF), N,N-dimethylacetamide (DMAc), THF, alcohols, chloroform, 1,4-dioxane, and mixtures thereof.

10. The process as claimed in claim 1, wherein the bioresorbable polymer is present in an amount of 5-50% weight fraction of the bioresorbable polymer solution.

11. The process as claimed in claim 10, wherein the bioresorbable polymer is present in an amount of 10-40% weight fraction of the bioresorbable polymer solution.

12. The process as claimed in claim 1, wherein the low molecular weight oligomer is present in an amount of 10-80% weight fraction based on the non-solvent portion of the bioresorbable polymer solution.

13. The process as claimed in claim 1, wherein the coagulant includes water and an organic solvent.

14. The process as claimed in claim 13, wherein the organic solvent in the coagulant is present in an amount of 5-60% weight fraction.

15. The process as claimed in claim 13, wherein the organic solvent in the coagulant is selected from the group consisting of amides, ketones, alcohols, and mixtures thereof.

16. The process as claimed in claim 15, wherein the organic solvent in the coagulant includes a ketone and an alcohol.

17. The process as claimed in claim 1, wherein the step of contacting the bioresorbable polymer solution with a coagulant is performed at a temperature of 5°C to 60°C.

18. The process as claimed in claim 17, wherein the step of contacting the bioresorbable polymer solution with a coagulant is performed at a temperature of 10°C to 50°C.

19. The process as claimed in claim 1, wherein after the bioresorbable polymer solution contacts the coagulant, the porous bioresorbable material is washed in a washing liquid.

20. The process as claimed in claim 19, wherein the washing liquid includes water and an organic solvent, wherein the organic solvent in the washing liquid is selected from the group consisting of ketones, alcohols, and mixtures thereof.

## Patentansprüche

1. Verfahren zur Herstellung eines porösen bioresorbierbaren Materials, in dem die Poren miteinander verbunden sind, umfassend die Schritte
Lösen eines bioresorbierbaren Polymers und eines niedermolekularen Oligomers in einem organischen Lösungsmittel zur Bildung einer Lösung des bioresorbierbaren Polymers, wobei das bioresorbierbare Polymer ein Molekulargewicht von mehr als 20 000 besitzt und das Oligomer ein Molekulargewicht von 200 bis 4000; und
Zusammenbringen der Lösung des bioresorbierbaren Polymers mit einem Koagulierungsmittel zur Bildung des porösen bioresorbierbaren Materials, wobei das niedermolekulare Oligomer in dem Koagulierungsmittel löslich und das bioresorbierbare Polymer in dem Koagulierungsmittel unlöslich ist.

2. Verfahren nach Anspruch 1, wobei vor dem Zusammenbringen der Lösung des bioresorbierbaren Polymers mit dem Koagulierungsmittel die Lösung in eine vorgegebene Form gebracht wird.

3. Verfahren nach Anspruch 2, wobei das Bringen der Lösung in eine vorgegebene Form das Schichten der Lösung auf eine Formoberfläche umfasst.

4. Verfahren nach Anspruch 2, wobei das Bringen der Lösung in eine vorgegebene Form das Gießen der Lösung in einen Behälter umfasst.

5. Verfahren nach Anspruch 1, wobei das bioresorbierbare Polymer ein Molekulargewicht von 20 000 bis 300 000 besitzt.

6. Verfahren nach Anspruch 1, wobei das bioresorbierbare Polymer ausgewählt ist aus der Gruppe Polycaprolacton (PCL), Polymilchsäure (PLA), Polyglycolsäure (PGA), Polymilchsäure-co-glycolsäure-Copolymer (PLGA-Copolymer), Polycaprolacton-Polymilchsäure-Copolymer (PCL-PLA-Copolymer), Polycaprolacton-Polyethylenglycol-Copolymer (PCL -PEG -Copolymer) und deren Gemische.

7. Verfahren nach Anspruch 1, wobei das niedermolekulare Oligomer ein Molekulargewicht von 300 bis 300 000 besitzt.

8. Verfahren nach Anspruch 1, wobei das niedermolekulare Oligomer ausgewählt ist aus der Gruppe Polycaprolactontriol (PCLTL), Polycaprolactondiol (PCLDL), Polycaprolacton (PCL), Polymilchsäure (PLA), Polyethylenglycol (PEG), Polypropylenglycol (PPG), Polytetramethylenglycol (PTMG) und deren Gemische.

9. Verfahren nach Anspruch 1, wobei das organische Lösungsmittel zum Lösen des bioresorbierbaren Polymers und des niedermolekularen Oligomers ausgewählt ist aus der Gruppe N,N-Dimethylformamid (DMF), N,N-Dimethylacetamid (DMAc), THF, Alkohole, Chloroform, 1,4-Dioxan und deren Gemische.

10. Verfahren nach Anspruch 1, wobei das bioresorbierbare Polymer in einer Menge von 5 bis 50 Gewichtsprozent der Lösung des bioresorbierbaren Polymers zugegen ist.

11. Verfahren nach Anspruch 10, wobei das bioresorbierbare Polymer in einer Menge von 10 bis 40 Gewichtsprozent der Lösung des bioresorbierbaren Polymers zugegen ist.

12. Verfahren nach Anspruch 1, wobei das niedermolekulare Oligomer in einer Menge von 10 bis 80 Gewichtsprozent zugegen ist, bezogen auf den Nicht-Lösungsmittelanteil der Lösung des bioresorbierbaren Polymers.

13. Verfahren nach Anspruch 1, wobei das Koagulierungsmittel Wasser und ein organisches Lösungsmittel enthält.

14. Verfahren nach Anspruch 13, wobei das organische Lösungsmittel in dem Koagulierungsmittel in einer Menge von 5 bis 60 Gewichtsprozent zugegen ist.

15. Verfahren nach Anspruch 13, wobei das organische Lösungsmittel in dem Koagulierungsmittel ausgewählt ist aus der Gruppe: Amide, Ketone, Alkohole und deren Gemische.

16. Verfahren nach Anspruch 15, wobei das organische Lösungsmittel in dem Koagulierungsmittel ein Keton und einen Alkohol umfasst..

17. Verfahren nach Anspruch 1, wobei das Zusammenbringen der Lösung des bioresorbierbaren Polymers mit einem Koagulierungsmittel bei einer Temperatur von 5 bis 60°C erfolgt.

18. Verfahren nach Anspruch 17, wobei das Zusammenbringen der Lösung des bioresorbierbaren Polymers mit einem Koagulierungsmittel bei einer Temperatur von 10 bis 50°C erfolgt.

19. Verfahren nach Anspruch 1, wobei nach dem Zusammenbringen der Lösung des bioresorbierbaren Polymers mit dem Koagulierungsmittel das poröse bioresorbierbare Material in einer Waschflüssigkeit gewaschen wird.

20. Verfahren nach Anspruch 19, wobei die Waschflüssigkeit Wasser und ein organisches Lösungsmittel enthält, wobei das organische Lösungsmittel in der Waschflüssigkeit ausgewählt ist aus der Gruppe Ketone, Alkohole und deren Gemische.

## Revendications

1. Procédé pour préparer une matière poreuse biorésorbable ayant des pores interconnectés, comprenant les étapes suivantes :
dissolution d'un polymère biorésorbable et un oligomère de faible poids moléculaire dans un solvant organique pour former une solution de polymère biorésorbable, dans lequel le polymère biorésorbable a un poids moléculaire supérieur à 20. 000 et l'oligomère a un poids moléculaire de 200 à 4000 ; et
mise en contact de la solution de polymère biorésorbable avec un coagulant pour former la matière poreuse biorésorbable, dans laquelle l'oligomère de faible poids moléculaire est soluble dans le coagulant, et le polymère biorésorbable est insoluble dans le coagulant.

2. Le procédé tel que revendiqué dans la revendication 1, dans lequel, avant que la solution de polymère biorésorbable soit mise en contact avec le coagulant, la solution est mise sous une forme prédéterminée.

3. Le procédé tel que revendiqué dans la revendication 2, dans lequel l'étape de mise sous une forme prédéterminée de la solution comprend l'application de la solution sous forme de revêtement sur la surface d'un moule.

4. Le procédé tel que revendiqué dans la revendication 2, dans lequel l'étape de mise sous une forme prédéterminée de la solution comprend la coulée de la solution dans un conteneur.

5. Le procédé tel que revendiqué dans la revendication 1, dans lequel le polymère biorésorbable a un poids moléculaire de 20 000 à 300 000.

6. Le procédé tel que revendiqué dans la revendication 1, dans lequel le polymère biorésorbable est choisi dans le groupe constitué par du polycaprolactone (PCL), de l'acide polylactique (PLA), l'acide polyglycolique (PGA), du copolymère de l'acide poly-lactique-co-glycolique (copolymère PLGA), du copolymère de l'acide polycaprolactone - polylactique (copolymère PCL - PLA), du copolymère de polycaprolactone - polyéthylène glycol (copolymère PCL-PEG) et les mélanges de ceux-ci.

7. Le procédé tel que revendiqué dans la revendication 1, dans lequel l'oligomère de faible poids moléculaire a un poids moléculaire de 300 à 3000.

8. Le procédé tel que revendiqué dans la revendication 1, dans lequel l'oligomère de faible poids moléculaire est choisi dans le groupe constitué par le polycaprolactone triol (PCLTL), le polycaprolactone diol (PCLDL), le polycaprolactone (PCL), l'acide polylactique (PLA), le polyéthylène glycol (PEG), le prolypropylène glycol (PPG), le polytétraméthylène glycol (PTMG), et les mélanges de ceux-ci.

9. Le procédé tel que revendiqué dans la revendication 1, dans lequel le solvant organique pour dissoudre le polymère biorésorbable et l'oligomère de faible poids moléculaire est choisi dans le groupe constitué par la N, N-diméthylformamide (DMF), la N, N-diméthylacétamide (DMAc), le THF, les alcools, le chloroforme, le 1,4 dioxane, et les mélanges de ceux-ci.

10. Le procédé tel que revendiqué dans la revendication 1, dans lequel le polymère biorésorbable est présent dans une quantité de 5-50% en poids de la solution de polymère biorésorbable.

11. Le procédé tel que revendiqué dans la revendication 10, dans lequel le polymère biorésorbable est présent dans une quantité de 10-40% en poids de la solution de polymère biorésorbable.

12. Le procédé tel que revendiqué dans la revendication 1, dans lequel l'oligomère à faible poids moléculaire est présent dans une quantité de 10-80% en poids basé sur la portion qui n'est pas le solvant de la solution de polymère biorésorbable.

13. Le procédé tel que revendiqué dans la revendication 1, dans lequel le coagulant comprend de l'eau et un solvant organique.

14. Le procédé tel que revendiqué dans la revendication 13, dans lequel le solvant organique est présent dans le coagulant dans une quantité de 5-60% en poids.

15. Le procédé tel que revendiqué dans la revendication 13, dans lequel le solvant organique du coagulant est choisi dans le groupe constitué par les amides, les cétones, les alcools, et les mélanges de ceux-ci.

16. Le procédé tel que revendiqué dans la revendication 15, dans lequel le solvant organique du coagulant comprend une cétone et un alcool.

17. Le procédé tel que revendiqué dans la revendication 1, dans lequel l'étape de mise en contact de la solution de polymère biorésorbable avec un coagulant est mise en oeuvre à une température de 5°C à 60°C.

18. Le procédé tel que revendiqué dans la revendication 17, dans lequel l'étape de mise en contact de la solution de polymère biorésorbable avec un coagulant est mise en oeuvre à une température de 10°C à 50°C.

19. Le procédé tel que revendiqué dans la revendication 1, dans lequel après mise en contact de la solution de polymère biorésorbable avec le coagulant, la matière poreuse biorésorbable est lavée dans un liquide de lavage.

20. Le procédé tel que revendiqué dans la revendication 19, dans lequel l'eau de lavage comprend de l'eau et un solvant organique, dans lequel le solvant organique du liquide de lavage est choisi dans le groupe constitué de cétones, d'alcools, et des mélanges de ceux-ci.
